# EUROPÄISCHE PATENTANMELDUNG

(11) **EP 4 560 313 A1**
(43) Veröffentlichungstag der Anmeldung: **28.05.2025**
(21) Anmeldenummer: 23211744.0
(22) Anmeldetag: 23.11.2023
(51) Int. Cl.: G01N 33/00

(54) **GASANALYSEVORRICHTUNG MIT VERBESSERTER TRENNANORDNUNG FÜR MOLEKULAREN WASSERSTOFF**

(71) Anmelder: Siemens Aktiengesellschaft, 80333 München (DE)
(72) Erfinder: Hörner, Thomas, 76133 Karlsruhe (DE)
(74) Vertreter: Siemens Patent Attorneys

(57) **Zusammenfassung**

Die Erfindung betrifft eine Gasanalysevorrichtung (10) zum Analysieren einer Stoffprobe (15), die molekularen Wasserstoff (16) enthält. Sie umfasst eine Trennanordnung (20) mit zumindest einer ersten Trennvorrichtung (21), die stromab mit einer Messvorrichtung (24) verbunden ist. Erfindungsgemäß ist die Gasanalysevorrichtung (10) mit einer ersten Trennvorrichtung (21) versehen, die eine erste Leitung (31) umfasst, die für molekularen Wasserstoff (16) zumindest teilweise permeabel und für einen Rest (18) der Stoffprobe (15) zumindest teilweise impermeabel ist. Die Messvorrichtung (24) ist zum Erfassen eines Massenstroms (27) und/oder Volumenstroms des molekularen Wasserstoffs (16) ausgebildet. Die Erfindung betrifft ebenso ein Verfahren (100) zum Erfassen einer Zusammensetzung einer Stoffprobe (15) mittels einer solchen Gasanalysevorrichtung (10) und eine erfindungsgemäße Verwendung von nicht-graphitischem Kohlenstoff. Ferner betrifft die Erfindung ein Verfahren (200) zum Simulieren eines Betriebsverhaltens eine derartigen Gasanalysevorrichtung (10) und ein dazu geeignetes Simulationsprogrammprodukt(60).

## Beschreibung

Die Erfindung betrifft eine Gasanalysevorrichtung zum Analysieren einer Stoffprobe, die molekularen Wasserstoff enthält. Die Erfindung betrifft ebenso ein Verfahren zum Ermitteln einer Zusammensetzung einer Stoffprobe, die molekularen Wasserstoff enthält. Weiter betrifft die Erfindung eine Verwendung von nicht-graphitischem Kohlenstoff, eine Simulationsverfahren und eine Simulationsprogrammprodukt.

Aus dem Artikel "Grüner Wasserstoff: Transport im Erdgasnetz", S. 113, Forschung kompakt, 01.04.2021, von der Fraunhofer-Gesellschaft ist eine Kohlenstoffbeschichtung bekannt, durch die Wasserstoff aus einem Gemisch mit Erdgas trennbar ist. Dazu wird das Erdgas-Wasserstoff-Gemisch in eine Mehrzahl an Leitungen eingeleitet, die in einem Rohr aufgenommen sind.

Die Druckschrift WO 2012/000727 A1 offenbart eine Vorrichtung zum Trennen von Gasen, die Membrantrennstufen umfasst. Dazu gehört eine Feedstromtrennstufe, die einen Feedstrom aus mindestens zwei Komponenten in einen ersten Permeatstrom und einen ersten Retentatstrom auftrennt. Eine Mehrzahl derartiger Membrantrennstufen ist darin miteinander verschaltet.

Gasanalysevorrichtungen werden zunehmend in Anwendungen eingesetzt, in denen Stoffproben zu analysieren sind, in denen molekularer Wasserstoff enthalten ist. Gleichzeitig bestehen hohe Anforderungen an die Sicherheit beim Betrieb von Gasanalysevorrichtungen. Es besteht somit Bedarf an einer Möglichkeit, die dazu geeignet ist, Stoffproben mit molekularem Wasserstoff präzise und sicher zu analysieren.

Die Aufgabenstellung wird durch eine erfindungsgemäße Gasanalysevorrichtung gelöst, die dazu ausgebildet ist, eine Stoffprobe zu analysieren, die molekularen Wasserstoff enthält. Die Gasanalysevorrichtung umfasst eine Trennanordnung, zu der zumindest eine erste Trennvorrichtung gehört. Die Trennanordnung ist im Betrieb von der Stoffprobe durchströmbar. Die Gasanalysevorrichtung ist stromab der Trennanordnung mit zumindest einer Messvorrichtung verbunden. Erfindungsgemäß umfasst die erste Trennvorrichtung zumindest eine erste Leitung, die für molekularen Wasserstoff zumindest teilweise permeabel ist. Die erste Leitung weist eine Wandung auf, durch die molekularer Wasserstoff durchtreten kann. Ebenso ist die erste Leitung für einen Rest der Stoffprobe, also alle anderen Komponenten der Stoffprobe, zumindest teilweise impermeabel. Die erste Leitung ist somit dazu ausgebildet, dass molekularer Wasserstoff bei einem Durchströmen der ersten Leitung aus dieser austritt. Korrespondierend ist die erste Leitung dazu ausgebildet, dass der Rest der Stoffprobe in der ersten Leitung verbleibt, wenn die Stoffprobe die erste Leitung durchströmt. Weiter ist die Messvorrichtung dazu ausgebildet und mit der Trennanordnung verbunden, einen Massenstrom und/oder Volumenstrom des molekularen Wasserstoffs zu erfassen, der durch die Trennanordnung aus der Stoffprobe separiert wird.

Die Trennanordnung der erfindungsgemäßen Gasanalysevorrichtung ist somit dazu geeignet, passiv den molekularen Wasserstoff von der Stoffprobe zu trennen. Dadurch sind aktive Komponenten, die beispielsweise elektrische Entladungen verursachen können, in der Trennanordnung entbehrlich. Das Separieren des molekularen Wasserstoffs ist im Wesentlichen durch einen Förderdruck angetrieben, mit dem die Stoffprobe der Trennanordnung zugeführt wird. Die Trennanordnung, und damit auch die erste Trennvorrichtung, sind dadurch besonders einfach und kosteneffizient herstellbar. Weiter sind ein Massenstrom und/oder ein Volumenstrom in einfacher Weise sicher und präzise messbar. Basierend auf dem gemessenen Massenstrom und/oder Volumenstrom des molekularen Wasserstoffs ist somit dessen Anteil an der Stoffprobe in einfacher Weise ermittelbar. Beispielsweise kann der Massenstrom und/oder Volumenstrom des Wasserstoffs auf eine Dosierangabe bezogen werden, durch die einen Massenstrom und/oder Volumenstrom der Stoffprobe bezogen werden, die der Trennanordnung im Betrieb zugeführt wird. Die Gasanalysevorrichtung kann hierzu mit einer Auswertungseinheit versehen sein, die zumindest mit der Messvorrichtung verbunden ist, mit der der Massenstrom und/oder Volumenstrom des molekularen Wasserstoffs erfassbar ist. Eine Messvorrichtung, mit der ein Massenstrom erfasst wird, kann dazu ausgebildet sein, einen absoluten Massenanteil des molekularen Wasserstoffs zu erfassen. Dazu kann die Messvorrichtung mit einer Druckreguliervorrichtung versehen sein. Alternativ oder ergänzend kann die Messvorrichtung als Rotameter ausgebildet sein. Mittels eines Rotameters ist der Volumenstrom des molekularen Wasserstoffs erfassbar. Eine derartige Messvorrichtung bietet ein erhöhtes Niveau an Explosionsschutz und erlaubt einen besonders sicheren Betrieb der Gasanalysevorrichtung.

In einer Ausführungsform der beanspruchten Gasanalysevorrichtung kann die erste Trennvorrichtung eine zweite Leitung umfassen, die zu einem Aufnehmen des durch die erste Leitung durchtretenden molekularen Wasserstoffs ausgebildet ist. Die zweite Leitung kann im Wesentlichen als Rohrleitung ausgebildet sein, in der die erste Leitung aufgenommen ist, und somit von der zweiten Leitung umgeben ist. Die zweite Leitung kann mit der Messvorrichtung verbunden sein, mit der der Massenstrom und/oder Volumenstrom des molekularen Wasserstoffs erfassbar ist. Die erste und zweite Leitung können derartige Innendurchmesser aufweisen, dass ein Zwischenraum zwischen der ersten und zweite Leitung minimiert ist. Je kleiner der Zwischenraum zwischen der ersten und zweiten Leitung ist, umso schneller folgt der Massenstrom bzw. Volumenstrom des molekularen Wasserstoffs einer Änderung des Anteils an molekularem Wasserstoff in der Stoffprobe. Leitungen, insbesondere Rohrleitungen, sind in einfacher Weise baulich anpassbar, um so den Anteil an molekularem Wasserstoff zu erfassen.

Weiter ist durch ein Anpassen der Durchmesser, also des der Innen- und Außendurchmesser der ersten und zweiten Leitung, die Oberfläche einstellbar, über die der molekulare Wasserstoff aus der ersten Leitung austritt. Die beanspruchte Gasanalysevorrichtung ist somit in einfacher Weise konstruktiv in puncto Trennverhalten anpassbar, und damit für eine breite Spanne an Einsatzzwecken geeignet. Weiter sind Rohrleitungen kosteneffizient in einer breiten Spanne an Abmessungen verfügbar, wodurch die Trennanordnung der beanspruchten Gasanalysevorrichtung wirtschaftlich herstellbar ist.

Des Weiteren kann die erste Leitung in ihrer Innenwand zumindest abschnittsweise mit einer Beschichtung beschichtet sein, die zumindest teilweise aus nicht-graphitischem Kohlenstoff hergestellt ist, insbesondere mit solchem, der eine turbostratische Struktur ausbildet. Die Beschichtung kann auch vollständig aus nicht-graphitischem Kohlenstoff, insbesondere solchem, der eine turbostratische Struktur ausbildet bzw. als turbostratische Struktur ausgebildet ist, hergestellt sein. Derartige Beschichtungen mit oder aus nicht-graphitischem Kohlenstoff sind für molekularen Wasserstoff zumindest teilweise permeabel und für Moleküle, die größer sind als molekularer Wasserstoff, im Wesentlichen impermeabel bzw. zumindest teilweise impermeabel. Weiter kann die Wandung der ersten Leitung zumindest teilweise aus einem Polymer, einer Keramik oder einer Kombination hieraus hergestellt sein. Dadurch ist insgesamt eine dauerhafte Filterwirkung gewährleistet, durch die der molekulare Wasserstoff wirksam von der verbleibenden Stoffprobe separierbar ist. Alternativ oder ergänzend kann die erste Leitung an ihrer Innenwand zumindest abschnittsweise mit einer Beschichtung beschichtet sein, die zumindest teilweise aus einem amorphen oder teilkristallinen Kunststoff hergestellt ist, beispielsweise Polyimid, Polyamid, Polyssulfon, Celluloseacetat und Derivate hiervor, Polyphenylenoxide, Polysiloxan, Polymer mit intrinsischer Mikroporosität, Mixed Matrix Membranen, Facilitated Transport Membranen, Polyethylenoxid, Polypropylenoxid oder Mischungen hieraus.

Darüber hinaus kann die Trennanordnung eine zweite Trennanordnung umfassen. Die zweite Trennanordnung ist seriell hinter der ersten Trennvorrichtung angeordnet oder kaskadierend zur ersten Trennvorrichtung angeordnet. Bei einer seriellen Anordnung der zweiten Trennvorrichtung wird dieser die verbleibende Stoffprobe aus der ersten Trennvorrichtung zugeführt. Die zweite Trennvorrichtung kann analog der ersten Trennvorrichtung eine erste Leitung umfassen, die von einer zweiten Leitung umgeben ist, und deren Innenwand zumindest abschnittsweise mit nicht-graphitischem Kohlenstoff beschichtet ist. Korrespondierend kann alternativ die erste Leitung der zweiten Trennvorrichtung an ihrer Innenwand zumindest abschnittsweise mit einer Beschichtung beschichtet sein, die zumindest teilweise aus einem amorphen oder teilkristallinen Kunststoff hergestellt ist, beispielsweise Polyimid, Polyamid, Polyssulfon, Celluloseacetat und Derivate hiervor, Polyphenylenoxide, Polysiloxan, Polymer mit intrinsischer Mikroporosität, Mixed Matrix Membranen, Facilitated Transport Membranen, Polyethylenoxid, Polypropylenoxid oder Mischungen hieraus. Weiter kann die zweite Trennvorrichtung in ihren Abmessungen, beispielsweise in puncto Innendurchmesser ihrer ersten Leitung, angepasst sein. Die aus der ersten Trennvorrichtung ausgeleitete verbleibende Stoffprobe weist einen reduzierten Anteil an molekularem Wasserstoff auf, an den die zweite Trennvorrichtung angepasst sein kann. Der durch die zweite Trennvorrichtung separierte molekulare Wasserstoff kann mit dem in der ersten Trennvorrichtung separiertem molekularen Wasserstoff zusammengeführt werden und der daraus resultierende Massenstrom und/oder Volumenstrom mit der Messvorrichtung erfasst werden. Die Messvorrichtung ist dabei entsprechend angeordnet, den kombinierten Massenstrom bzw. Volumenstrom an molekularem Wasserstoff zu erfassen. Durch einen solchen seriellen Aufbau der Trennanordnung wird eine gesteigerte Trennwirkung erreicht und der Anteil an molekularem Wasserstoff im Rest der Stoffprobe reduziert, die aus der zweiten Trennvorrichtung ausleitbar ist. Dadurch wird die Betriebssicherheit der Gasanalysevorrichtung, insbesondere bei einer weiteren Analyse der verbleibenden Stoffprobe, weiter gesteigert.

Alternativ oder ergänzend kann die zweite Trennvorrichtung kaskadierend mit der ersten Trennvorrichtung verbunden sein. Bei der kaskadierenden Anordnung wird der in der ersten Trennvorrichtung separierte molekulare Wasserstoff der zweiten Trennvorrichtung zugeführt. Die zweite Trennvorrichtung kann hierbei, wie bei der seriellen Anordnung, korrespondierend zur ersten Trennvorrichtung ausgebildet sein. In der kaskadierenden Anordnung der zweiten Trennvorrichtung werden Fremdkomponenten aus einer Strömung mit überwiegend molekularem Wasserstoff separiert. Hierdurch wird die Reinheit des molekularen Wasserstoffs gesteigert, zu dem der Massenstrom und/oder Volumenstrom zu erfassen ist. Insbesondere bei einer Erfassung des Massenstroms wird so eine Verfälschung des Messergebnisses vorgebeugt. Insbesondere wird vermieden, dass ein scheinbar erhöhter Massenstrom vorliegt. Bei der kaskadierenden Anordnung der ersten und zweiten Trennvorrichtung können die in der zweiten Trennvorrichtung verbleibenden Fremdkomponenten mit der verbleibenden Stoffprobe, die aus der ersten Trennvorrichtung ausgeleitet wird, zusammengeführt werden. Durch die kaskadierende Anordnung der ersten und zweiten Trennvorrichtung wird insgesamt eine gesteigerte Messgenauigkeit beim Erfassen des Anteils an molekularem Wasserstoff in der Stoffprobe erreicht. Darüber hinaus können die serielle und kaskadierende Anordnung unter Einbeziehung zumindest einer dritten Trennvorrichtung kombiniert werden. Hierdurch ist die Trennwirksamkeit der Trennanordnung in einfacher Weise an den jeweiligen Anwendungsfall skalierbar anpassbar.

In einer weiteren Ausführungsform der beanspruchten Gasanalysevorrichtung kann die erste Leitung zumindest einer der Trennvorrichtungen als Mehrfachleitung ausgebildet sein. Die erste Leitung kann dazu eine Mehrzahl an geschlossenen Leitungsquerschnitten aufweisen, die ein Bündel von Leitungen bilden. Die erste Leitung kann als Mehrfachleitung eine Mehrzahl an Leitungsabschnitten aufweisen, die im Wesentlichen parallel zueinander angeordnet sind. Die geschlossenen Leitungsquerschnitte können beispielsweise im Wesentlichen kreisförmig ausgebildet sein. Durch eine derartige Mehrfachleitung ist ein Leitungsquerschnitt der zweiten Leitung stärker ausfüllbar, die die erste Leitung umgibt. Ferner ist so eine Oberfläche vergrößert, durch die molekularer Wasserstoff aus der ersten Leitung in die zweite Leitung durchtreten kann. Hierdurch wird das Separieren des molekularen Wasserstoffs von der Stoffprobe beschleunigt. Dadurch ist ein Anstieg des Anteils an molekularem Wasserstoff in der Stoffprobe beschleunigt erkennbar. Dies erlaubt die Verwendung der beanspruchten Gasanalysevorrichtung in sicherheitskritischen Anwendungen, beispielsweise bei der Überwachung eines Elektrolyseurs oder eines empfindlichen chemischen Produktionsprozesses.

Darüber hinaus kann die erste Leitung in der beanspruchten Gasanalysevorrichtung mit einer weiteren Messvorrichtung versehen sein, also mit ihr verbunden sein, die zu einem Erfassen eines Massenstroms und/oder Volumenstroms des Rests der Stoffprobe ausgebildet ist. Der Rest der Stoffprobe bzw. die verbleibende Stoffprobe entspricht hierbei der der Trennanordnung zugeführten Stoffprobe, von der der darin enthaltene molekulare Wasserstoff separiert ist. In Verbindung mit der Messvorrichtung, die mit der zweiten Leitung verbunden ist, ist somit durch Vergleich der Massenströme bzw. Volumenströme von molekularem Wasserstoff und dem Rest der Stoffprobe der Anteil an molekularem Wasserstoff in der Stoffprobe bestimmbar. Bei einer Änderung der Zusammensetzung der Stoffprobe, also bei veränderlichem Anteil an molekularem Wasserstoff, folgen die Massenströme bzw. Volumenströme schnell der Änderung der Zusammensetzung. Dementsprechend ist beispielsweise ein schneller Anstieg des Anteils an molekularem Wasserstoff frühzeitig erkennbar. Dadurch ist die beanspruchte Gasanalysevorrichtung auch zur Überwachung von sicherheitskritischen Anwendungen geeignet. Alternativ oder ergänzend kann auch eine weitere Messvorrichtung zufuhrseitig mit der Trennanordnung verbunden sein und dazu ausgebildet sein, einen Gesamtmassenstrom der Stoffprobe zu erfassen. Der Anteil an molekularem Wasserstoff in der Stoffprobe ist dementsprechend durch Bezug auf den Gesamtmassenstrom zu ermitteln.

Ferner kann die beanspruchte Gasanalysevorrichtung mit einem Detektor versehen sein, der zu einem Analysieren der verbleibenden Stoffprobe aus der Trennanordnung ausgebildet ist. Der Detektor kann dazu mittelbar oder unmittelbar mit mindestens einer der Leitungen der ersten und/oder zweiten Trennvorrichtung verbunden sein. Der Detektor kann beispielweise als Flammenionisationsdetektor, als Wärmeleitfähigkeitsdetektor, als Photoionisationsdetektor, als flammenphotometrischer Detektor, als Elektroneneinfangdetektor, als Atomemissionsdetektor, als Plasmadetektor, als Massenspektrometer, Ionen-Mobilitäts-Spektrometer und/oder Massenspektrometer ausgebildet sein. Des Weiteren kann die Gasanalysevorrichtung mit zumindest einer Trennsäule versehen sein, die beispielsweise stromab der Trennanordnung angebracht sein kann. Die Trennsäule ist weiter vorzugsweise stromauf des Detektors positioniert. Durch ein Separieren von molekularem Wasserstoff aus der Stoffprobe ist die verbleibende Stoffprobe in vorteilhafter Weise in Detektoren einsetzbar, in denen die Stoffprobe entzündet wird oder einer Energieentladung ausgesetzt wird. Folglich sind Stoffproben mit molekularem Wasserstoff mit der beanspruchten Gasanalysevorrichtung sicher handhabbar. Ferner ist die beanspruchte Gasanalysevorrichtung mit einer breiten Spanne an Analytik-Konzepten kombinierbar und ist dadurch vielseitig einsetzbar.

Die eingangs beschriebene Aufgabenstellung wird ebenso durch ein erfindungsgemäßes Verfahren zum Ermitteln einer Zusammensetzung einer Stoffprobe gelöst, die molekularen Wasserstoff enthält. Hierzu wird eine Gasanalysevorrichtung eingesetzt. Das Verfahren umfasst einen ersten Schritt, in dem die Stoffprobe in eine erste Leitung zugeführt wird, die zu einer Trennanordnung der Gasanalysevorrichtung gehört. Die Trennanordnung ist dazu ausgebildet, den molekularen Wasserstoff von der Stoffprobe zu separieren. Weiter gehört ein zweiter Schritt zum Verfahren, in dem der molekulare Wasserstoff gesammelt und abgeführt wird, der eine Wandung der ersten Leitung durchdringt. Die erste Leitung ist für molekularen Wasserstoff zumindest teilweise permeabel und für den Rest der Stoffprobe, also die verbleibende Stoffprobe, zumindest teilweise impermeabel. Die verbleibende Stoffprobe wird im zweiten Schritt des Verfahrens weitergeleitet und beispielsweise aus der Trennanordnung ausgeleitet.

Des Weiteren umfasst das Verfahren einen dritten Schritt, in dem ein Massenstrom und/oder Volumenstrom des abgeführten molekularen Wasserstoffs erfasst wird. Der molekulare Wasserstoff wird hierzu einer geeigneten Messvorrichtung zugeführt. Basierend auf dem erfassten Massenstrom und/oder Volumenstrom wird weiter im dritten Schritt ein Wasserstoffanteil an der Stoffprobe ermittelt, die im ersten Schritt zugeführt wird.

Erfindungsgemäß ist die erste Leitung zumindest abschnittsweise an ihrer Innenwandung mit nicht-graphitischem Kohlenstoff beschichtet, insbesondere mit nicht-graphitischem Kohlenstoff, der turbostratische Strukturen ausbildet bzw. als turbostratische Struktur ausgebildet ist. Durch die Beschichtung mit nicht-graphitischem Kohlenstoff ist gewährleistet, dass lediglich der molekulare Wasserstoff aus der ersten Leitung austritt und der im zweiten Schritt gesammelte und abgeführte molekulare Wasserstoff eine erhöhte Reinheit aufweist. Ebenso ist so gewährleitet, dass die verbleibende Stoffprobe, also der Rest der Stoffprobe, im Wesentlichen frei von molekularem Wasserstoff ist. Dies ermöglicht ein einfaches und gleichzeitig präzises Erfassen des Wasserstoffanteils, also des Anteils an molekularem Wasserstoff, in der Stoffprobe. Weiter sind Massenströme bzw. Volumenströme auch in molekularem Wasserstoff bei relativ niedrigen Temperaturen und ohne signifikante Energieentladungen präzise erfassbar. Hierdurch wird die Betriebssicherheit beim erfindungsgemäßen Verfahren gesteigert. Im erfindungsgemäßen Verfahren kann die Stoffprobe im Wesentlichen durch einen Förderdruck durch die Trennanordnung geführt werden, die dementsprechend aus passiven Komponenten hergestellt sein kann. Unter einer passiven Komponente ist hierbei jegliche Komponente zu verstehen, die frei von einer Energieversorgung betreibbar ist. Durch das Separieren des molekularen Wasserstoffs vom Rest der Stoffprobe wird deren Handhabung ebenso weiter vereinfacht.

In einer Ausführungsform des beanspruchten Verfahrens kann die Gasanalysevorrichtung, auf der das Verfahren durchgeführt wird, gemäß einer der oben skizzierten Ausführungsformen ausgebildet sein. Die Merkmale der Gasanalysevorrichtung gelten analog auch für das beanspruchte Verfahren. Insbesondere kann der Rest der Stoffprobe, also die verbleibende Stoffprobe, einer Trennsäule zugeführt werden, hinter der wiederum ein Detektor angeordnet ist. In einem vierten Verfahrensschritt kann somit der Rest der Stoffprobe auf seine Zusammensetzung analysiert werden. Die technischen Vorzüge der beanspruchten Gasanalysevorrichtung gelten insgesamt auch für das beanspruchte Verfahren.

Die oben skizzierte Aufgabe wird auch durch eine erfindungsgemäße Verwendung von nicht-graphitischem Kohlenstoff, insbesondere turbostratischem Kohlenstoff, gelöst. Der nicht-graphitische Kohlenstoff wird zu einem Separieren von molekularem Wasserstoff aus einer Stoffprobe eingesetzt, die eine erste Leitung durchströmt. Der nicht-graphitische Kohlenstoff ist als Beschichtung an einer Innenwand der ersten Leitung angebracht. Erfindungsgemäß gehört die erste Leitung zu einer Trennanordnung einer Gasanalysevorrichtung. Die Gasanalysevorrichtung ist dazu ausgebildet, eine Zusammensetzung der Stoffprobe zu ermitteln. Die Erfindung beruht unter anderem auf der überraschenden Erkenntnis, dass eine Beschichtung mit nicht-graphitischem Kohlenstoff eine derart sortenreine Separation des molekularen Wasserstoffs in der Stoffprobe erlaubt, dass dessen Anteil mit einer Präzision messbar ist, die großtechnischen Zwecken, sondern auch analytischen Zwecken genügt. Die erfindungsgemäße Verwendung von nicht-graphitischem Kohlenstoff erlaubt es somit, Gasanalysevorrichtungen, die wasserstoffhaltige Stoffproben analysieren, in puncto Sicherheit zu verbessern und konstruktiv zu vereinfachen.

In einer Ausführungsform der beanspruchten Verwendung ist die Gasanalysevorrichtung, zu der die erste Leitung gehört, die mit dem nicht-graphitischen Kohlenstoff beschichtet ist, als eine Gasanalysevorrichtung nach einer der oben skizzierten Ausführungsformen ausgebildet. Die Merkmale der Gasanalysevorrichtung sind somit auf die beanspruchte Verwendung übertragbar und erzielen die gleichen technischen Vorzüge.

Ferner wird die eingangs beschriebene Aufgabenstellung durch ein erfindungsgemäßes Verfahren zum Simulieren eines Betriebsverhaltens einer Gasanalysevorrichtung gelöst. Das Verfahren umfasst einen ersten Schritt, in dem ein Datensatz bereitgestellt wird, durch den zumindest die erste Leitung zumindest abschnittsweise nachgestellt ist. Der Datensatz kann einen Aufbau der ersten Leitung, also deren Geometrie und/oder Materialeigenschaften umfassen. Der Datensatz stellt insgesamt eine virtuelle Repräsentanz zumindest der ersten Leitung dar. Weiter umfasst das erfindungsgemäße Verfahren einen zweiten Schritt, in dem zumindest ein Betriebsparameter vorgegeben wird, durch den das zu simulierende Betriebsverhalten definiert ist. Das Vorgeben des Betriebsparameters kann durch einen Benutzer des Verfahrens erfolgen, einen Algorithmus, und/oder ein weiteres simulationsgerichtetes Computerprogramm. Durch den Betriebsparameter kann beispielsweise eine Zusammensetzung einer Stoffprobe vorgegeben sein, die durch die Gasanalysevorrichtung zu analysieren ist, deren Dosierung, also deren Menge, deren Temperatur, Druck und/oder Strömungsgeschwindigkeit. Ebenso kann der zumindest eine Betriebsparameter eine Angabe über Umgebungsbedingungen umfassen, beispielsweise eine Umgebungstemperatur und oder einen Umgebungsdruck.

Des Weiteren gehört ein dritter Schritt zum Verfahren, in dem ein Simulationsprogrammprodukt ausgeführt wird, das dazu ausgebildet ist, basierend auf dem Datensatz aus dem ersten Schritt und dem zumindest einen Betriebsparameter aus dem zweiten Schritt einen Leistungsparameter der simulierten Gasanalysevorrichtung zu ermitteln. Der Leistungsparameter kann hierbei jegliche Größe sein, die sich durch das Durchströmen der ersten Leitung durch die Stoffprobe ergibt. Der Leistungsparameter kann beispielsweise eine Menge an molekularem Wasserstoff sein, die die Wandung der ersten Leitung durchdringt und/oder eine Dauer, bis ein vorgebbarer Grad an Separation zwischen dem molekularen Wasserstoff und der verbleibenden Stoffprobe eintritt. Beim Ausführen des Simulationsprogrammprodukts wird eine virtuelle Repräsentanz der Stoffprobe mit dem molekularen Wasserstoff bereitgestellt, die zumindest die erste Leitung, also ihre virtuelle Repräsentanz, durchströmt. Im dritten Schritt des Verfahrens wird weiter der zumindest eine Leistungsparameter durch das Simulationsprogrammprodukt ermittelt. Ferner gehört ein vierter Schritt zum Verfahren, in dem der im dritten Schritt ermittelte Leistungsparameter an einen Benutzer und/oder eine Datenschnittstelle ausgegeben wird. Die Datenschnittstelle kann dazu ausgebildet sein, den Datensatz, den zumindest einen Betriebsparameter und/oder den zumindest einen Leistungsparameter an ein weiteres simulationsgerichtetes Computerprogramm weiterzugeben.

Erfindungsgemäß ist die Gasanalysevorrichtung, deren Betriebsverhalten simuliert wird, gemäß einer der oben beschriebenen Ausführungsformen ausgebildet. Der Erfindung liegt unter anderem die überraschende Erkenntnis zugrunde, dass ein Permeationsverhalten von molekularem Wasserstoff durch die Wandung der ersten Leitung in einfacher Weise schnell und präzise simulierbar ist. Eine Permeation von anderen Bestandteilen der Stoffprobe ist vernachlässigbar, wodurch der Simulationsaufwand reduziert ist. Weiter ist die Verteilung des molekularen Wasserstoffs in einem Leitungsquerschnitt in der Stoffprobe als gleichmäßige Verteilung darstellbar. Dadurch lässt sich beispielsweise eine Rotationssymmetrie der ersten Leitung nutzen. Das Permeationsverhalten in einem Leitungsquerschnitt ist im Wesentlichen als eindimensionales Verhalten modellierbar. Durch Aneinanderreihung mehrerer Leitungsquerschnitte entlang einer Leitungsachse, die durch die Mittelpunkte der Leitungsquerschnitte verläuft, ist das Permeationsverhalten des molekularen Wasserstoffs vereinfacht simulierbar. Das Permeationsverhalten des Wasserstoffs durch die Wandung der ersten Leitung ist anhand einer vorgebbaren Anzahl an Leitungsquerschnitten entlang der ersten Leitung nachstellbar. Die Anzahl an berücksichtigten Leitungsquerschnitten ist hierbei reduzierbar, da das Permeationsverhalten zwischen zwei Leitungsquerschnitten mit erhöhter Genauigkeit interpolierbar ist. Dementsprechend ist das Betriebsverhalten der ersten Leitung, und damit der Gasanalysevorrichtung, präzise und schnell simulierbar. Insbesondere ist dadurch Echtzeitfähigkeit für das erfindungsgemäße Verfahren erzielbar. Folglich ist das erfindungsgemäße Verfahren dazu geeignet, den Betrieb der Gasanalysevorrichtung betriebsbegleitend zu überwachen. Dadurch sind Beschädigungen von Komponenten der Gasanalysevorrichtung, beispielsweise der ersten Leitung, frühzeitig erkennbar, was einen sicheren Betrieb der Gasanalysevorrichtung ermöglicht.

Die oben dargelegte Aufgabe durch ein erfindungsgemäßes Simulationsprogrammprodukt gelöst. Das Simulationsprogrammprodukt ist zum Simulieren eines Betriebsverhaltens einer Gasanalysevorrichtung ausgebildet. Erfindungsgemäß ist das Simulationprogrammprodukt dazu ausgebildet, ein Verfahren, also ein Simulationsverfahren, nach einer der oben skizzierten Ausführungsformen umzusetzen. Das Simulationsprogrammprodukt kann dazu als Digitaler Zwilling der Gasanalysevorrichtung ausgebildet sein, wie beispielsweise in der Druckschrift US 2017/286572 A1 näher beschrieben. Der Offenbarungsgehalt von US 2017/286572 A1 wird durch Verweisung in die vorliegende Anmeldung miteinbezogen. Das Simulationsprogrammprodukt kann ein Physik-Modul umfassen, das dazu ausgebildet ist, ein Permeationsverhalten eines Gases durch einen Festkörper, beispielsweise eine Wandung, nachzustellen. Alternativ oder ergänzend kann das Physik-Modul dazu ausgebildet sein, ein Strömungsverhalten eines Gases in einer Leitung nachzustellen. Das Simulationsprogrammprodukt kann monolithisch ausgebildet sein, also vollständig auf einer Hardwareplattform ausführbar sein. Alternativ kann das Simulationsprogrammprodukt modular ausgebildet sein, also eine Mehrzahl an Teilprogrammen umfassen, die auf separaten Hardwareplattformen ausführbar sind und über eine kommunikative Datenverbindung zusammenwirken. Eine solche kommunikative Datenverbindung kann eine Netzwerkverbindung, eine Internetverbindung und/oder eine Mobilfunkverbindung sein.

Die Erfindung wird im Folgenden anhand einzelner Ausführungsformen in Figuren näher erläutert. Die Figuren sind insoweit in gegenseitiger Ergänzung zu lesen, dass gleiche Bezugszeichen in unterschiedlichen Figuren die gleiche technische Bedeutung haben. Ferner sind die einzelnen Merkmale der in den Figuren gezeigten Ausführungsformen auch untereinander und mit den oben skizzierten Merkmalen kombinierbar. Es zeigen im Einzelnen:
- FIG 1: einen schematischen Aufbau einer Ausführungsform der beanspruchten Gasanalysevorrichtung;
- FIG 2: einen schematischen Ausschnitt aus einem Aufbau einer ersten Ausführungsform der ersten Trennvorrichtung;
- FIG 3: eine schematische Darstellung der ersten Ausführungsform der ersten Trennvorrichtung im Längsschnitt;
- FIG 4: eine schematische Darstellung einer zweiten Ausführungsform der ersten Trennvorrichtung in einer Schrägansicht.

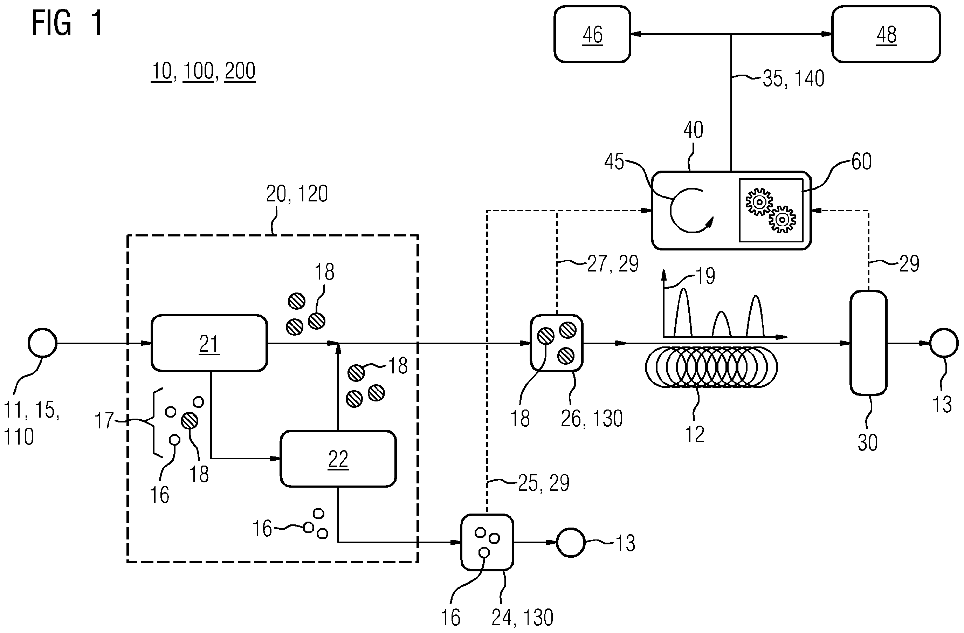

In FIG 1 ist schematisch ein Aufbau einer Ausführungsform der beanspruchten Gasanalysevorrichtung 10 gezeigt. Ebenso ist schematisch der Ablauf eines Verfahrens 100 zum Erfassen einer Zusammensetzung einer Stoffprobe 15 dargestellt, das auf der Gasanalysevorrichtung durchgeführt wird. Die Gasanalysevorrichtung 10 weist einen Gaseinlass 11 auf, über den die Stoffprobe 15 zuführbar ist. Der Gaseinlass 11 ist mit einer Trennanordnung 20 verbunden, die dazu ausgebildet ist, aus der Stoffprobe 15 molekularen Wasserstoff 16 zu separieren. Die Trennanordnung 20 umfasst eine erste Trennvorrichtung 21 und eine zweite Trennvorrichtung 22, die kaskadierend miteinander verbunden sind. Die erste und zweite Trennvorrichtung 21, 22 sind im Wesentlichen baugleich ausgebildet. Die erste Trennvorrichtung 21 ist dazu ausgebildet, aus der Stoffprobe 15 molekularen Wasserstoff 16 zu separieren. Der von der ersten Trennvorrichtung 21 separierte molekulare Wasserstoff 16 ist mit den Rest 18 der Stoffprobe 15 versetzt und bildet ein Zwischenfiltrat 17. Dieses wird der zweiten Trennvorrichtung 22 zugeführt. Korrespondierend ist die zweite Trennvorrichtung 22 dazu ausgebildet, im Wesentlichen reinen molekularen Wasserstoff 16 zu separieren. Reste 18 der Stoffprobe 15 aus der ersten und zweiten Trennvorrichtung 21, 22 werden zusammengeführt. Dementsprechend sind der molekulare Wasserstoff 16 und der Rest 18 der Stoffprobe 15 separat aus der Trennanordnung 20 ausleitbar.

Die Trennanordnung 20 ist derart mit einer Messvorrichtung 24 verbunden, dass damit der molekulare Wasserstoff 16 aus der Trennanordnung 20 in puncto Massenstrom und/oder Volumenstrom erfassbar ist. Korrespondierend ist die Trennanordnung 20 derart mit einer weiteren Messvorrichtung 26 verbunden, dass damit der Massenstrom und/oder Volumenstrom des Rests 18 der Stoffprobe 15 erfassbar ist. Die Messvorrichtungen 24, 26 sind ferner mit eine Auswertungseinheit 40 verbunden, die zu einem Auswerten von erfassten Massenströmen 25, 27 von molekularem Wasserstoff 16 und dem Rest 18 der Stoffprobe 15. Dazu werden geeignete Messsignale 29 an die Auswertungseinheit 40 gesendet. Das Vergleichen zwischen den Massenstrom 25 an molekularem Wasserstoff 16 und dem Massenstrom 27 des Rest 18 der Stoffprobe 15 ist der Anteil des molekularen Wasserstoffs 16 an der Stoffprobe 15 ermittelbar. Die Auswertungseinheit 40 ist hierzu mit einem geeigneten Computerprogrammprodukt 45 ausgestattet.

Weiter verfügt die Gasanalysevorrichtung 10 über eine Trennsäule 12, die der Trennanordnung 20 und der weiteren Messvorrichtung 26 nachgeschaltet ist, also stromab von diesen liegt. Der Trennsäule 12 ist der Rest 18 der Stoffprobe 15 zuführbar und durch Retention in ihre Komponenten trennbar. Das Trennen des Rests 18 der Stoffprobe 15 ist durch ein Chromatogramm 19 symbolisiert. Ferner ist ein Detektor 30 mit der Trennsäule 12 verbunden, der dazu ausgebildet ist, die Komponenten des Rests 18 der Stoffprobe 15 zu erkennen und/oder zu quantifizieren. Zur Auswertung der Messungen des Detektors 30 ist dieser mit der Auswertungseinheit 40 über eine kommunikative Datenverbindung verbunden, über die entsprechende Messignale 2 übertragbar sind. Stromab des Detektors 30 ist der Rest 18 der Stoffprobe 15 über einen Gasaulass 13 aus der Gasanalysevorrichtung 10 abführbar. Analog ist der molekulare Wasserstoff 16 über einen Gasauslass 13 stromab der Messvorrichtung 24 aus der Gasanalysevorrichtung 10 abführbar.

Die Auswertungseinheit 40 ist weiter mit einer Anzeigeeinheit 46 und einer Datenschnittstelle 48 verbunden. Darüber sind Resultate über die Erfassung der Zusammensetzung der Stoffprobe 15 an einen Benutzer bzw. an ein weiteres Computerprogrammprodukt übertragbar. Aus der Auswertungseinheit 20 ist ferner ein Simulationsprogrammprodukt 60 ausführbar gespeichert, das als Digitaler Zwilling zumindest der Trennanordnung 20 ausgebildet ist und mit dem der Betrieb der Gasanalysevorrichtung 10 überwachbar ist. Das Simulationsprogrammprodukt 60 ist zu diesem Zweck dazu ausgebildet, ein nicht näher gezeigtes Simulationsverfahren 200 durchzuführen.

Mit der dargestellten Gasanalysevorrichtung 10 ist ein Verfahren 100 zum Ermitteln einer Zusammensetzung der Stoffprobe 15 durchführbar. In einem ersten Schritt 110 des Verfahren 100 wird die Stoffprobe 15 über den Gaseinlass 11 zugeführt und zur Trennanordnung 20 geleitet. In der Trennanordnung 20 wird die Stoffprobe 15 in einem zweiten Schritt 120 im Wesentlichen in molekularen Wasserstoff 16 und den Rest 18 der Stoffprobe 15 separiert. Es folgt ein dritter Schritt 130, in dem der Massenstrom 25 des molekularen Wasserstoffs 16 mittels einer Messvorrichtung 24 erfasst wird. Ebenso wird im dritten Schritt 130 ein Massenstrom 27 des Rest 18 der Stoffprobe 15 mit einer weiteren Messvorrichtung 26 erfasst. Gleichermaßen wird im dritten Schritt 130 basierend auf den Massenströmen 25, 27 der Anteil 35 an molekularem Wasserstoff 16 in der Stoffprobe 15 ermittelt. Dazu wird das Computerprogrammprodukt 45 verwendet, das auf der Auswertungseinheit 40 ausführbar gespeichert ist. Der ermittelte Anteil 35 an molekularem Wasserstoff 16 in der Stoffprobe 15 wird über die Anzeigeeinheit 46 und/oder die Datenschnittstelle 48 ausgegeben.

Der Aufbau der Trennvorrichtung 20, die die zugrundeliegende Separation des molekularen Wasserstoffs 16 aus der Stoffprobe 15 ermöglicht, wird im Folgenden detaillierter dargestellt und beschrieben.

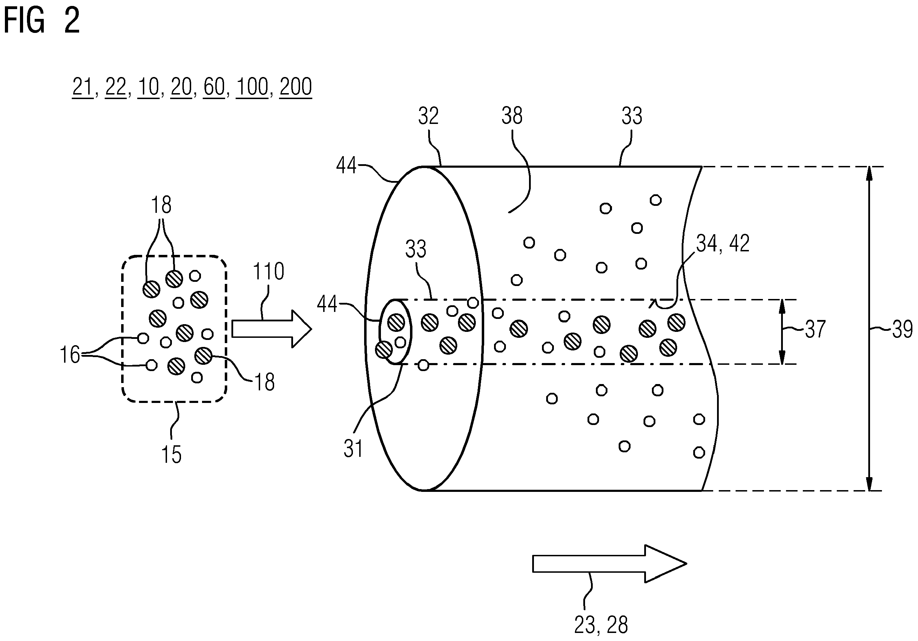

FIG 2 zeigt schematisch einen Ausschnitt aus einem Aufbau einer ersten Ausführungsform der ersten Trennvorrichtung 21, die in einer Gasanalysevorrichtung 10, wie in FIG 1 gezeigt, einsetzbar ist. Korrespondierend zur ersten Trennvorrichtung 21 kann auch deren zweite Trennvorrichtung 22 ausgebildet sein oder auch jegliche weitere Trennvorrichtung in einer Trennanordnung 20 in einer Gasanalysevorrichtung 10 wie in FIG 1. Mittels der gezeigten ersten Trennvorrichtung 21 ist auch ein Verfahren 100, wie in FIG 1 dargestellt, umsetzbar.

Die erste Trennanordnung 21 umfasst zumindest eine erste Leitung 31, die von einer zweiten Leitung 32 umgeben ist. Die erste und zweite Leitung 31, 32 weisen jeweils eine Wandung 33 auf und sind im Wesentlichen rohrförmig. Ebenso weisen die ersten und zweite Leitung 31, 32 einen im Wesentlichen kreisförmigen Leitungsquerschnitt 44 auf. Der Innendurchmesser 37 der ersten Leitung 31 ist geringer als der Innendurchmesser 39 der zweiten Leitung 39. Die erste Leitung 31 ist somit von der zweiten Leitung 32 umgeben und zwischen diesen ist ein Zwischenraum 38 ausgebildet. Der ersten Leitung 31 ist im Betrieb der ersten Trennvorrichtung 21 eine Stoffprobe 15 zugeführt, die molekularen Wasserstoff 16 und einen Rest 18 der Stoffprobe 15 umfasst. Der Rest 18 der Stoffprobe 15 kann eine Mehrzahl an Komponenten umfassen. Die Stoffprobe 15 wird im ersten Schritt 110 des Verfahrens 100, wie in FIG 1 gezeigt, der ersten Leitung 31, zugeführt. Die erste Leitung 31 in an ihrer Innenwand 34 zumindest teilweise mit einer Beschichtung 42 versehen, die zumindest teilweise aus nicht-graphitischem Kohlenstoff, insbesondere aus solchem, der eine turbostratische Struktur ausbildet bzw. als turbostratische Struktur ausgebildet ist, hergestellt ist. Die Beschichtung 42 ist für den molekularen Wasserstoff 16 im Wesentlichen permeabel, so dass der molekulare Wasserstoff 16 die Wandung 33 der ersten Leitung 31 durchdringt, also durch die Wandung 33 in den Zwischenraum 38 durchtritt. Die Beschichtung 42 ist für den Rest 18 der Stoffprobe 15 im Wesentlichen impermeabel. Entlang einer Strömungsrichtung 28 verringert sich die Menge an molekularem Wasserstoff 16 in der Stoffprobe 15 in der ersten Leitung 31 somit. Korrespondierend steigt die Menge an molekularem Wasserstoff 16 in der zweiten Leitung 32. Die Strömung der Stoffprobe 15, also des molekularen Wasserstoffs 16 und der Rests 18 wird durch einen Förderdruck 23 angetrieben, mit dem die Stoffprobe 15 der entsprechenden Trennanordnung 20 zugeführt wird. Der molekulare Wasserstoff 16 in der zweiten Leitung 32 ist aus dem Zwischenraum 38, also der zweiten Leitung 32, separat abführbar. Ebenso ist der verbleibende Rest 18 der Stoffprobe 15 separat aus der ersten Leitung 31 abführbar. Für den molekularen Wasserstoff 16 und den Rest 18 der Stoffprobe 15 sind so durch separate Messvorrichtung 24, 26, wie in FIG 1 exemplarisch gezeigt, deren jeweiliger Massenstrom und/oder Volumenstrom erfassbar.

FIG 2 zeigt zu besseren Übersicht einen idealisierten Trennvorgang. In einer Ausführungsform wie beispielsweise in FIG 1 wird zumindest in der ersten Trennvorrichtung 21 keine ideale Trennung erzielt. Vielmehr ist mit Beimengungen des Rests 18 der Stoffprobe 15 im molekularen Wasserstoff 16 zu rechnen.

Zumindest die erste Leitung 31 ist als virtuelle Repräsentanz in einem Simulationsprogrammprodukt 60 als Datensatz hinterlegt. Das Simulationsprogrammprodukt 60 ist als Digitaler Zwilling zumindest der ersten Leitung 31 ausgebildet und dazu eingerichtet, deren Betriebsverhalten im Zuge eines nicht näher gezeigten Simulationsverfahrens 200 nachzustellen.

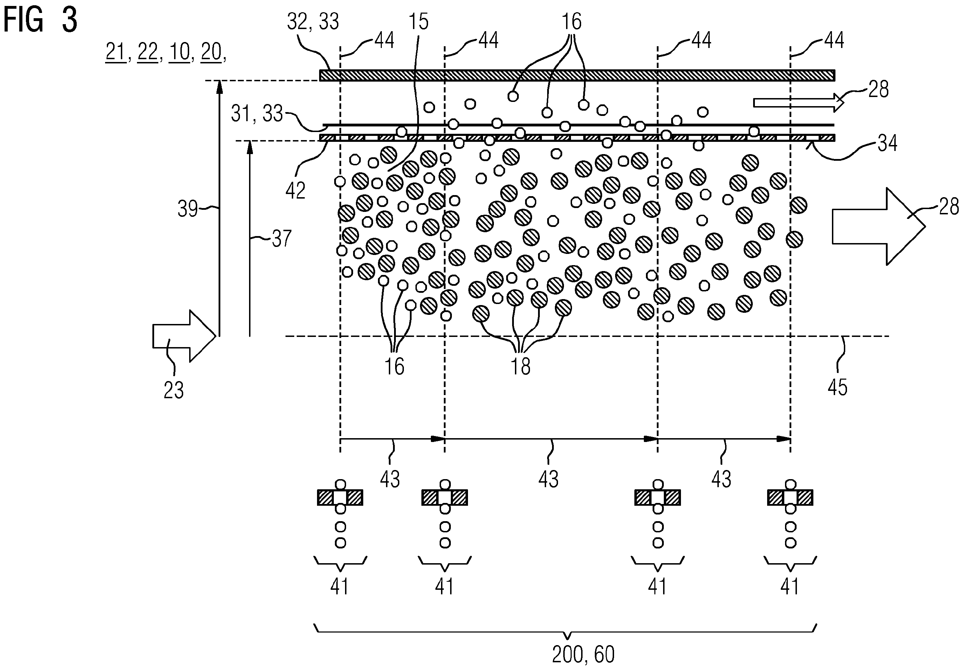

Die erste Ausführungsform der ersten Trennvorrichtung 21 gemäß FIG 2 ist in FIG 3 schematisch in einem Längsschnitt ausschnittsweise dargestellt. Die erste Trennvorrichtung 21 gehört zur Trennanordnung 20 einer Gasanalysevorrichtung 10. Eine zweite Trennvorrichtung 22 der Trennanordnung 20 kann korrespondierend zur gezeigten ersten Trennvorrichtung 21 ausgebildet sein. Die in FIG 3 gezeigten Merkmale gelten somit analog auch für eine zweite Trennvorrichtung 22.

Die erste Trennvorrichtung 21 umfasst die erste Leitung 31, die von der zweiten Leitung 32 umgeben ist. Der ersten Leitung 31 wird durch einen Förderdruck 23 die Stoffprobe 15 entlang einer Leitungsachse 45 zugeführt, die molekularen Wasserstoff 16 und einen Rest 18 aufweist, wobei der Rest 18 mehrere Komponenten, also unterschiedliche Stoffe, umfassen kann. Die Innenseite der Wandung 33 der ersten Leitung 31, also deren Innenwand 34, ist abschnittsweise zumindest teilweise mit der Beschichtung 42 versehen, die zumindest teilweise aus nicht-graphitischem Kohlenstoff hergestellt ist. Die Beschichtung 42 ist für den molekularen Wasserstoff 16 zumindest teilweise permeabel und für den Rest 18 der Stoffprobe 15 im Wesentlichen impermeabel. Beim Durchströmen der ersten Leitung 31 tritt der molekulare Wasserstoff 16 durch die Beschichtung 42 und die Wandung 33 der ersten Leitung hindurch und tritt in einen Zwischenraum 38 ein, der durch die Wandungen 33 der ersten und zweiten Leitung 31, 32 definiert ist. Entlang der Leitungsachse 45 nimmt die Menge an molekularem Wasserstoff 16 in der ersten Leitung 31 ab und in der zweiten Leitung 32, also im Zwischenraum 38, korrespondierend zu. Der Innendurchmesser 37 der ersten Leitung 31 und der Innendurchmesser 39 der zweiten Leitung 32 sind, unter Berücksichtigung der Wandstärke der Wandung 33 der ersten Leitung 31, derart dimensioniert, dass eine wirksame Abfuhr des darin eingetretenen molekularen Wasserstoffs 16 gewährleistet ist. Die erste Leitung 31 ist derart dimensioniert, dass die Innenwand 34 mit der Beschichtung 42 eine maximierte Oberfläche aufweist, wodurch das Separieren des molekularen Wasserstoffs 16 vom Rest 18 der Stoffprobe 15 unterstützt wird. Durch den Förderdruck 23 ist die Abfuhr des Rests 18 der Stoffprobe 15 und des molekularen Wasserstoffs 16 entlang der Strömungsrichtung 28 gewährleistet.

FIG 3 zeigt zur besseren Übersicht, korrespondierend zu FIG 2, einen idealisierten Trennvorgang. In einer Ausführungsform wie beispielsweise in FIG 1 wird zumindest in der ersten Trennvorrichtung 21 keine ideale Trennung erzielt. Vielmehr ist mit Beimengungen des Rests 18 der Stoffprobe 15 im molekularen Wasserstoff 16 zu rechnen.

Das Separationsverhalten des molekularen Wasserstoffs 16 vom Rest 18 der Stoffprobe 15 wird in einem Simulationsverfahren 200 nachgestellt, das mittels des Simulationsprogrammprodukts 60 durchgeführt wird. Das Simulationsprogrammprodukt 60 umfasst einen Datensatz, in dem der Aufbau zumindest der ersten Leitung 31 abgebildet ist. Als Betriebsparameter sind der Förderdruck 23, die Menge an zugeführter Stoffprobe 15 und deren Zusammensetzung vorgebbar. Als Leistungsparameter sind mit dem Simulationsprogrammprodukt 60 eine Menge und/oder eine Reinheit des molekularen Wasserstoffs 16 ermittelbar, der aus dem Zwischenraum 38, also der zweiten Leitung 32, abgeführt wird. Alternativ oder ergänzend sind mittels des Simulationsprogrammprodukts 60 Menge und/oder eine Reinheit des Rests 18 der Stoffprobe 15 ermittelbar, die aus der ersten Leistung 31 abgeführt wird. Dazu wird das Permeationsverhalten 41 des molekularen Wasserstoffs 16 durch die Wandung 33 der ersten Leitung 31 mit der Beschichtung 42 an einer Mehrzahl an Leitungsquerschnitten 44 nachgestellt. Das Permeationsverhalten 41 ist je Leitungsquerschnitts 44 als eindimensionales Permeationsverhalten 41 nachgestellt. Da der Rest 18 der Stoffprobe 15 aus größeren Molekülen als Wasserstoffmolekülen besteht, ist die Beschichtung 42 für den Rest 18 im Wesentlichen impermeabel, so dass eine Simulation des Permeationsverhaltens des Rests 18 entbehrlich ist. Dadurch, dass die erste Leitung 31 im Wesentlichen rotationssymmetrisch ausgebildet ist, ist das eindimensional nachgestellte Permeationsverhalten 41 in einfacher Weise auf den gesamten Leitungsquerschnitt 44 übertragbar. Zur Simulation des Permeationsverhaltens 41 entlang der ersten Leitung 31 in Strömungsrichtung 28 wird dieses für eine Mehrzahl an beabstandeten Leitungsquerschnitten 44 ermittelt, die als Stützstellen dienen. Zwischen den entsprechenden Leitungsquerschnitten 44 erfolgt eine Interpolation 43, durch die in den Abschnitten zwischen den Leitungsquerschnitten 44 das Permeationsverhalten 41 hinreichend präzise nachstellbar ist. Das Simulationsprogrammprodukt 60 ist folglich dazu geeignet, das Betriebsverhalten der ersten Trennvorrichtung 21 mit reduziertem Rechenaufwand schnell nachzustellen, wodurch sich für eine breite Spanne an Anwendungsfällen Echtzeitfähigkeit für das entsprechende Simulationsverfahren 200 ergibt.

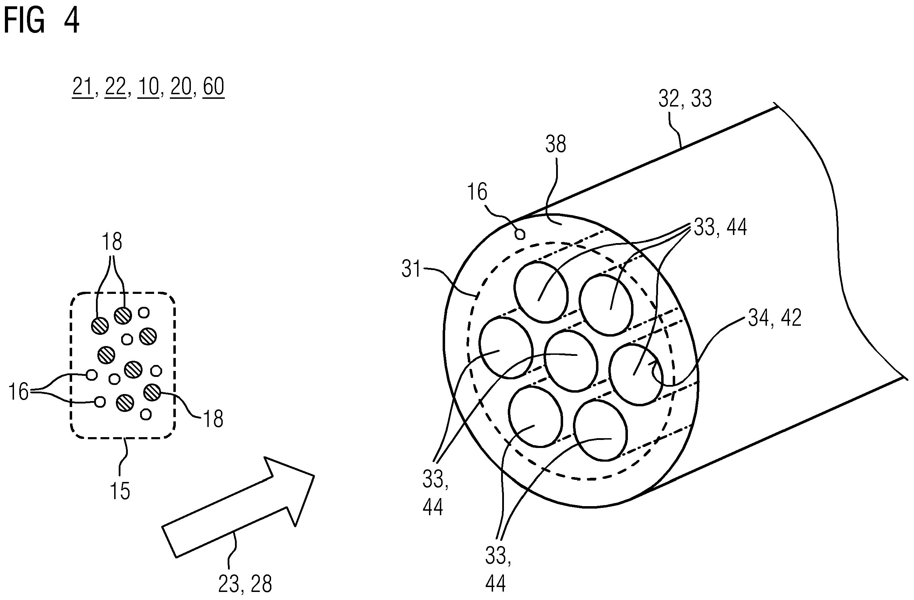

Eine erste Trennvorrichtung 21 gemäß einer zweiten Ausführungsform der beanspruchten Gasanalysevorrichtung 10 ist in FIG 4 schematisch in einer Schrägansicht gezeigt. Die erste Trennvorrichtung 21 gehört zu einer Trennanordnung 20. Eine zweite Trennvorrichtung 22 der Trennanordnung 20 kann korrespondierend zur ersten Trennvorrichtung 21 ausgebildet sein.

Die erste Trennvorrichtung 21 umfasst eine Mehrzahl erste Leitung 31, die als Mehrfachleitung ausgebildet ist. Die erste Leitung 31 umfasst eine Mehrzahl an im Wesentlichen kreisförmigen Leitungsquerschnitten 44, die jeweils durch eine Wandung 33 begrenzt sind. Die Mehrfachleitung ist im Wesentlichen bündelförmig ausgebildet, so dass eine Oberfläche von Innenwänden 34 mit einer Beschichtung 42 maximiert ist.

Die Einzelleitungen der Mehrfachleitung können baugleich ausgebildet sein oder bautypenverschieden. Dadurch ist die Mehrfachleitung an eine breite Spanne von Anwendungsfällen anpassbar, beispielsweise durch ein Anpassen der Durchmesser der Einzelleitungen. Die Beschichtung 42 ist hierbei zumindest teilweise aus einem nicht-graphitischen Kohlenstoff hergestellt, insbesondere einem solchen, der turbostratische Strukturen ausbildet bzw. als turbostratische Struktur ausgebildet ist. Der Aufbau der einzelnen Leitungsquerschnitte 44 kann jeweils einem Aufbau wie in FIG 3 entsprechen. Die als Mehrfachleitung ausgebildete erste Leitung 31 ist von einer zweiten Leitung 32 umgeben. Zwischen den Leitungsquerschnitten 44 der ersten Leitung 31 und der zweiten Leitung 32 ist ein Zwischenraum 38 ausgebildet, durch den separierter molekularer Wasserstoff 16 abführbar ist. Die erste Leitung 31 gemäß FIG 4 ist in einem Simulationsprogrammprodukt 60 abgebildet, das als Digitaler Zwilling ausgebildet ist und mit dem ein Simulationsverfahren 200 durchführbar ist, mit dem das Betriebsverhalten der zugehörigen Gasanalysevorrichtung 10 nachstellbar ist.

## Patentansprüche

1. Gasanalysevorrichtung (10) zum Analysieren einer Stoffprobe (15), die molekularen Wasserstoff (16) enthält, umfassend eine Trennanordnung (20) mit zumindest einer ersten Trennvorrichtung (21), die stromab mit einer Messvorrichtung (24) verbunden ist, **dadurch gekennzeichnet, dass** die erste Trennvorrichtung (21) eine erste Leitung (31) umfasst, die für molekularen Wasserstoff (16) zumindest teilweise permeabel und für einen Rest (18) der Stoffprobe (15) zumindest teilweise impermeabel ist, wobei die Messvorrichtung (24) zum Erfassen eines Massenstroms (27) und/oder Volumenstroms des molekularen Wasserstoffs (16) ausgebildet ist.

2. Gasanalysevorrichtung (10) nach Anspruch 1, **dadurch gekennzeichnet, dass** die erste Trennvorrichtung (21) eine zweite Leitung (32) umfasst, die zu einem Aufnehmen des durch die erste Leitung (31) durchtretenden molekularen Wasserstoffs (16) ausgebildet ist.

3. Gasanalysevorrichtung (10) nach Anspruch 1 oder 2, **dadurch gekennzeichnet, dass** die erste Leitung (31) an ihrer Innenwand (34) zumindest abschnittsweise einer Beschichtung (42) beschichtet ist, die zumindest teilweise aus nicht-graphitischem Kohlenstoff hergestellt ist.

4. Gasanalysevorrichtung (10) nach einem der Ansprüche 1 bis 3, **dadurch gekennzeichnet, dass** der nicht-graphitische Kohlenstoff eine turbostratische Struktur ausbildet.

5. Gasanalysevorrichtung (10) nach einem der Ansprüche 1 bis 4, **dadurch gekennzeichnet, dass** die Trennanordnung (20) eine zweite Trennvorrichtung (22) umfasst, die seriell oder kaskadierend mit der ersten Trennvorrichtung (21) verbunden ist.

6. Gasanalysevorrichtung (10) nach einem der Ansprüche 1 bis 5, **dadurch gekennzeichnet, dass** die erste Leitung (31) als Mehrfachleitung ausgebildet ist.

7. Gasanalysevorrichtung (10) nach einem der Ansprüche 1 bis 6, **dadurch gekennzeichnet, dass** die erste Leitung (31) mit einer weiteren Messvorrichtung (26) versehen ist, die zu einem Erfassen eines Massenstroms (27) und/oder Volumenstroms eines Rests (18) der Stoffprobe (15) ausgebildet ist.

8. Gasanalysevorrichtung (10) nach einem der Ansprüche 1 bis 7, **dadurch gekennzeichnet, dass** die Gasanalysevorrichtung (10) mit einem Detektor (30) versehen ist.

9. Verfahren (100) zum Ermitteln einer Zusammensetzung einer Stoffprobe (15), die molekularen Wasserstoff (16) enthält, mittels einer Gasanalysevorrichtung (10), umfassend die Schritte:
a) Zuführen der Stoffprobe (15) in eine erste Leitung (31) einer Trennanordnung (20) der Gasanalysevorrichtung (10);
b) Sammeln und Abführen des molekularen Wasserstoffs (16), der eine Wandung (33) der ersten Leitung (31) durchdringt und Weiterleiten der verbleibenden Stoffprobe (15);
c) Erfassen eines Massenstroms (27) und/oder Volumenstroms des abgeführten molekularen Wasserstoffs (16) und basierend hierauf Ermitteln eines Wasserstoffanteils (35) an der zugeführten Stoffprobe (15);
wobei die erste Leitung (31) zumindest abschnittsweise an ihrer Innenwand (34) mit nicht-graphitischem Kohlenstoff beschichtet ist.

10. Verfahren (100) nach Anspruch 8, **dadurch gekennzeichnet, dass** die Gasanalysevorrichtung (10) gemäß einem der Ansprüche 1 bis 8 ausgebildet ist.

11. Verwendung von nicht-graphitischem Kohlenstoff, insbesondere Kohlenstoff, der eine turbostratische Struktur ausbildet, zu einem Separieren von molekularem Wasserstoff (16) aus einer Stoffprobe (15), als Beschichtung (42) einer ersten Leitung (31), **dadurch gekennzeichnet, dass** die erste Leitung (31) zu einer Trennanordnung (20) einer Gasanalysevorrichtung (10) gehört.

12. Verwendung von nicht graphitischem Kohlenstoff nach Anspruch 10, **dadurch gekennzeichnet, dass** die Gasanalysevorrichtung (10) nach einem der Ansprüche 1 bis 8 ausgebildet ist.

13. Verfahren (200) zum Simulieren eines Betriebsverhaltens einer Gasanalysevorrichtung (10), umfassend die Schritte:
a) Bereitstellen eines Datensatzes, durch den zumindest die erste Leitung (31) zumindest abschnittsweise nachgestellt ist;
b) Vorgeben zumindest eines Betriebsparameters, durch den das zu simulierende Betriebsverhalten definiert ist;
c) Ausführen eines Simulationsprogrammprodukts (60), das dazu ausgebildet ist, basierend auf dem Datensatz und dem zumindest einen Betriebsparameter einen Leistungsparameter der simulierten Gasanalysevorrichtung (10) zu ermitteln und Ermitteln des Leistungsparameters;
d) Ausgeben des ermittelten Leistungsparameters an einen Benutzer und/oder eine Datenschnittstelle;
**dadurch gekennzeichnet, dass** die Gasanalysevorrichtung (10) gemäß einem der Ansprüche 1 bis 8 ausgebildet ist.

14. Simulationsprogrammprodukt (60) zum Simulieren eines Betriebsverhaltens einer Gasanalysevorrichtung (10), **dadurch gekennzeichnet, dass** das Simulationsprogrammprodukt (60) dazu ausgebildet ist, ein Verfahren (200) nach Anspruch 13 durchzuführen.
